# EUROPEAN PATENT SPECIFICATION

(11) **EP 0 500 791 B1**
(45) Date of publication and mention of the grant of the patent: **04.02.1998**
(21) Application number: 91900636.1
(22) Date of filing: 31.10.1990
(51) Int. Cl.: A61K 39/21, C07K 7/04

(54) **THE DESIGN AND CONSTRUCTION OF NON-INFECTIOUS HUMAN RETROVIRAL MUTANTS DEFICIENT IN GENOMIC RNA**
ENTWURF UND KONSTRUKTION VON NICHTINFEKTIÖSEN MENSCHLICHEN RETROVIRUSMUTANTEN MIT MANGEL AN GENOMISCHER RNA
CONCEPTION ET CONSTRUCTION DES MUTANTS RETROVIRAUX HUMAINS ET NON INFECTIEUX DEFICIENTS DANS L'ARN GENOMIQUE

(30) Priority: 31.10.1989 US 429287
(43) Date of publication of application: 02.09.1992
(73) Proprietor: THE UNITED STATES OF AMERICA as represented by the Secretary UNITED STATES DEPARTMENT OF COMMERCE, Washington, DC 20231 (US)
(72) Inventor: GORELICK, Robert, J., Braddock Heights, MD 21714-0291 (US); REIN, Alan, R., Takoma Park, MD 20912 (US); ARTHUR, Larry, O., Walkersville, MD 21793 (US); HENDERSON, Louis, E., Mount Airy, MD 21771 (US); OROSZLAN, Stephen, Potomac, MD 20854 (US)
(74) Representative: Jump, Timothy John Simon
(86) International application number: US9006231
(87) International publication number: WO9106318

(56) References cited:
- WO-A-91/05860
- PROCEEDINGS OF THE NATL. ACADEMY OF SCIENCES USA, vol. 85, no. 22, November 1988, Washington, DC (US); R.J. GORELICK et al., pp. 8420-8424
- JOURNAL OF VIROLOGY, vol. 63, no. 4, April 1989; C. MERIC et al., pp. 1558-1568
- JOURNAL OF VIROLOGY, vol. 64, no. 5, May 1990; A. ALDOVINI et al., pp. 1920- 1926
- CELL, vol. 33, May 1983; MANN et al., pp. 153-159
- JOURNAL OF VIROLOGY, vol. 64, July 1990; GORELICK et al., pp. 3207-3211
- JOURNAL OF BIOLOGICAL CHEMISTRY, vol. 256, no. 16, 25 August 1981; HENDERSON et al., pp. 8400-8406
- NUCLEIC ACIDS RESEARCH, vol. 14, no. 2, 1986; COVEY, pp. 632-633
- SCIENCE, vol. 232, 25 April 1986; BERG, pp. 485-487

## Description

The present invention relates to the disruption of vital protein structures common to all retroviruses and involved in the selection and packaging of genomic viral DNA into infectious virus particles. More specifically, the present invention relates to the drastic reduction or complete elimination of the infectivity of human immunodeficiency virus 1 (HIV-1), by the generation of mutants that lack some or all of the invariant residues required to form the structure.

A virus particle is a "package" in which virus-coded proteins surround and protect the viral genome. Thus, the viral proteins must be able to specifically recognize and select the viral genome for "packaging" during virus assembly. Very little is known about the mechanisms involved in this recognition, although in the case of retroviruses, a noncoding sequence near the 5' end of the viral RNA is necessary (P. Shank et al (1980) J. Virol. 36, 450-456; R. Mann et al (1983) Cell 33, 153-159) but not sufficient (J. Sorge et al (1983) J. Virol. 48, 667-675; S. Goff et al (1987) Biochem. Biophys. Acta 907, 93-123) for efficient encapsidation of the RNA. One naturally occurring mutant of avian sarcoma virus which has apparently lost the ability to selectively encapsidate viral RNA has been described (M. Linial et al (1978) Cell 15, 1371-1381), but the lesion in the viral proteins responsible for this defect has not yet been identified.

All retroviruses encode a polyprotein, termed the *gag* precursor, which plays a critical role in the virus assembly process. After virus assembly and release have occurred, the polyprotein is cleaved into the *gag* or core proteins found in the nature, infectious virus particle. One of these proteins, the "nucleocapsid" protein, binds nucleic acids in vitro and is believed to be associated with the genomic RNA in the virion. In all retroviruses, the nucleocapsid protein contains either one or two copies of the sequence:
-Cys-X-X-Cys-X-X-X-X-His-X-X-X-X-Cys-
(L. Henderson et al (1981) J. Biol. Chem. 256, 8400-8406; S. Oroszlan et al (1985) Current Topics in Microbiology and Immunology, P. Vogt, ed., Springer-Verlag, New York, pp. 221-233; S. Covey (1986) Nucl. Acids Res. 14, 623-633). As previously described (J. Berg (1986) Science 232, 485-486), this sequence bears a striking resemblance to the "zinc finger" protein sequences implicated in recognition of specific DNA sequences by a wide variety of eukaryotic transcription factors (R. Evans et al (1988) Cell 52, 1-3). However, zinc fingers have not been shown to interact with RNA.

The ubiquity of this motif in retroviral nucleocapsid proteins suggests that it performs an essential function in the viral life cycle, and its similarity to the "zinc fingers" raises the possibility that it is involved in the specific interaction with the viral genome which occurs during virus assembly. Accordingly, the present inventors tested the effects of mutations in this motif upon the infectivity of the virus and upon its ability to package genomic RNA, as reported in Proc. Natl. Acad. Sci., U.S.A, 1988, Vol.85, 8420-8424. The results of these experiments, as set forth below, show that the motif is indeed involved in specific RNA recognition and that mutations in the motif destroy the ability of the viral proteins to specifically package viral RNA during virus assembly. In International Patent Application Number WO9105860 Young et al. disclose point mutants of Moloney murine leukaemia virus in which the cysteine residues at positions 1 and 4 of the motif have been changed to tyrosine residues. Young et al. confirm that the mutations result in the inability to package viral RNA and suggest that the mutant viral particles so produced are still able to generate an immune response.

However, up until now, it has not been shown that mutants other than those disclosed by Young et al. are both non-infective and capable of generating an immune response, thus rendering them suitable for use as vaccines.

It is an object of the present invention to define a biological role for the following sequence of amino acids that is found in the nucleocapsid domain of the *gag* precursor polyproteins of all replication-competent retroviruses:
-Cys-X-X-Cys-X-X-X-X-His-X-X-X-X-Cys-
wherein X represents variable amino acids. The invariant residues constitute part of a vital protein structure, at least one of which are found in all retroviruses and which are involved in the selection and packaging of genomic viral RNA into infectious virus particles. Disruption of this structure leads to the formation of virus-like particles which appear to be structurally normal, but which do not contain the normal complement of viral RNA. Therefore, their infectivity is drastically reduced or completely eliminated.

It is thus another object of the present invention to drastically reduce or eliminate the infectivity of human immunodeficiency virus 1 (HIV-1), by generating mutants that lack some or all of the invariant residues required to form the structure.

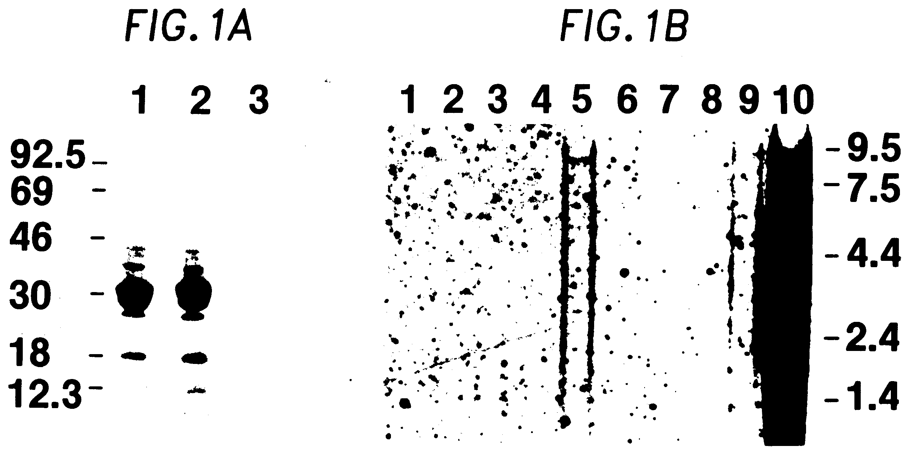

FIGURE 1A and FIGURE 1B show the properties of viral mutants. Figure 1A shows the protein immunoblotting. Virus was harvested from one 150 cm² flask containing 35 ml media and processed for protein immunoblotting, using antiserum against p30^{gag}. Lanes: 1, mutant C39S; 2, wild type Mo-MuLV; 3, negative control, pGCcos3neo. Protein molecular size markers (in kilodaltons) are indicated. Figure 1B shows the hybridization of viral RNA. Virus samples were harvested and adjusted for equal amounts of p30^{gag}. RNA was isolated from virus pellets and hybridized to a Mo-MuLV *gag-pol* probe (extending from Xho I [nucleotide 1560 (T. Shinnick et al (1981) Nature (London) 293, 543-548)] to *Sal* I [nucleotide 3705]). Lanes: 1, negative control, pGCcos3neo; 2, mutant C26S; 3, mutant C26S/C29S; 4, mutant C29S; 5, mutant Y28S; 6, mutant W35S; 7, mutant C39S; 8 and 9, 1:100 and 1:10 dilution of wild type Mo-MuLV, respectively; 10, wild type Mo-MuLV. RNA molecular size markers (in kilobases) are indicated.

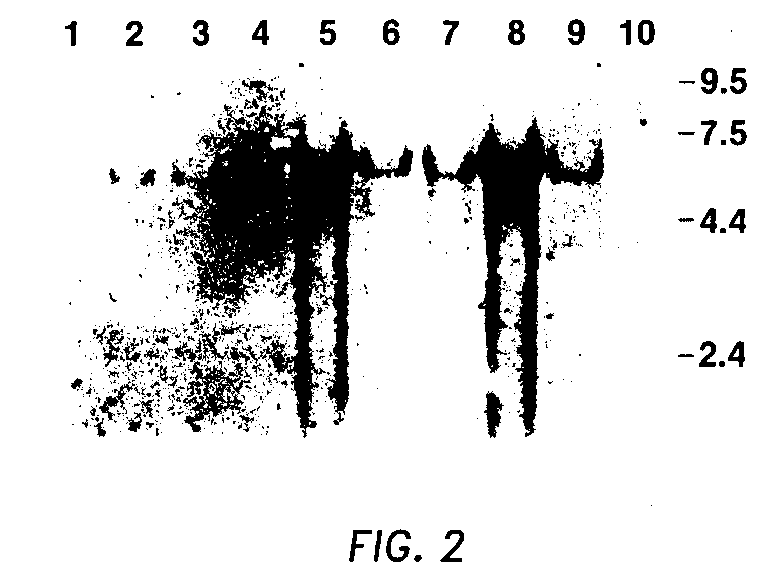

FIGURE 2 shows the hybridization of RNA from virus contained in culture fluids from KiSV transformed rat cells. K-NRK cells were transfected with plasmids containing mutant or wild type Mo-MuLV genomes, and stable transfectants were selected with G-418. Virus samples were harvested and adjusted for equal amounts of p30^{gag}. RNA was isolated from virus pellets and hybridized to a 1 kilobase *Pst* I-*Sal* I Kirsten-*ras* probe (M. McCoy et al (1984) Mol. Cell. Biol. 4, 1577-1582). Lanes: 1, negative control, pGCcos3neo; 2, mutant C26S; 3, mutant C26S/C29S: 4, mutant C29S; 5, mutant Y28S; 6, mutant W35S; 7, mutant C39S; 8, wild type Mo-MuLV: 9 and 10, 1:10 and 1:100 dilution of wild type Mo-MuLV, respectively. RNA molecular size markers (in kilobases) are indicated.

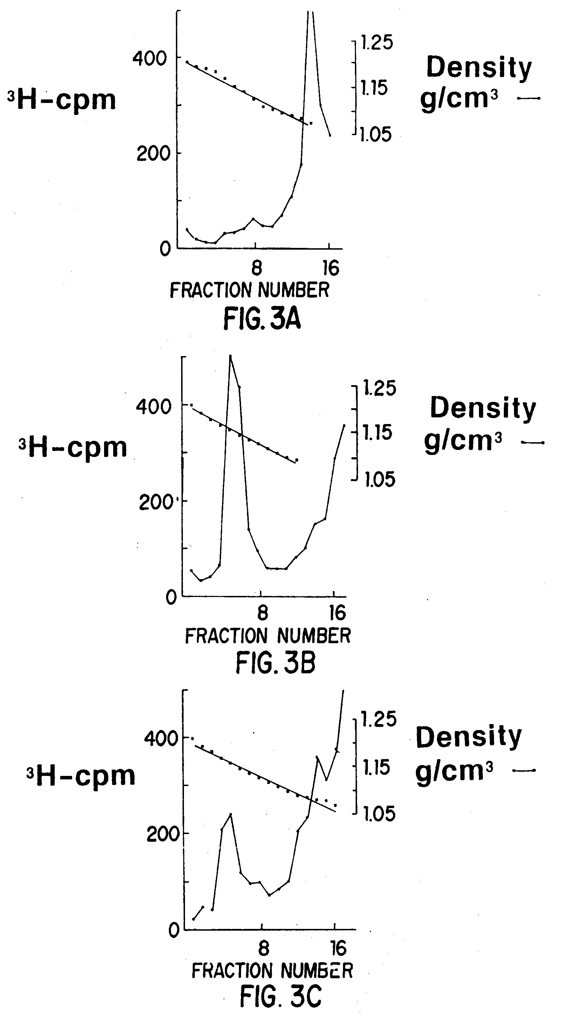

FIGURE 3A, FIGURE 3B and FIGURE 3C show the ³H-uridine counts of sucrose banded mutants and wild type virus. Cells containing mutant or wild type viral constructs in 150 cm² flasks were labeled with 4 ml of media containing 0.1 mCi/ml[5-³H]-uridine for 12 hours, and virus was harvested by centrifugation. For each sample, virus from three flasks were layered on a 10-50% sucrose gradient in 100 mM NaCl, 10 mM TRIS, pH 7.4, 1 mM EDTA. Gradients were centrifuged for 36 hr in a Beckman SW-41 rotor at 40,000 RPM, 4°C. ∼1 ml fractions were removed from the bottom of each sample. (-□-) density of fraction in g/cm³. (-o-) ³H-CPM of fraction. Figure 3A: negative control, pGCcos3neo. Figure 3B: wild type Mo-MuLV. Figure 3C: mutant C26S.

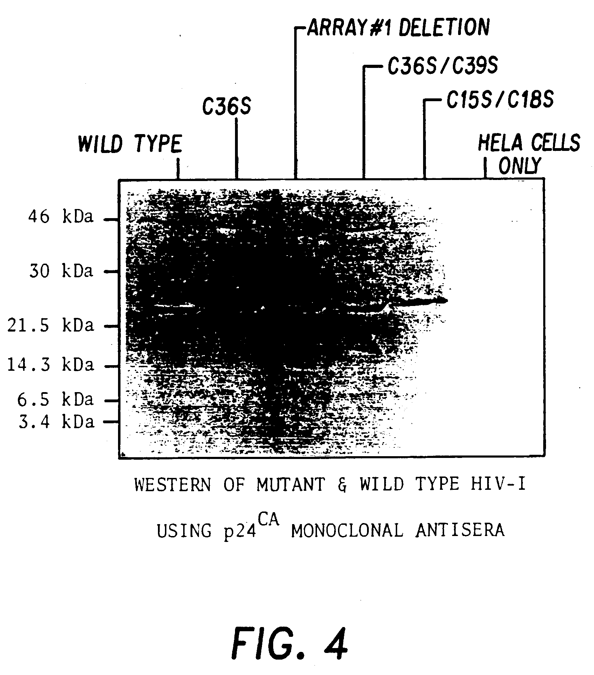

FIGURE 4 shows protein immunoblotting of mutant and wild type HIV-I using p24 CA monoclonal antibody. Hela cells were transfected with mutant and wild type viral clones. Four days later, clarified culture fluids were pelleted through a 1.0 mL cushion of 20% sucrose in phosphate buffered saline. Samples were adjusted for equal amounts of reverse transcriptase activities and processed for protein immunoblotting. The p24 CA monoclonal antibody complex was visualized using horseradish peroxidase staining. Samples are as labeled on the figure.

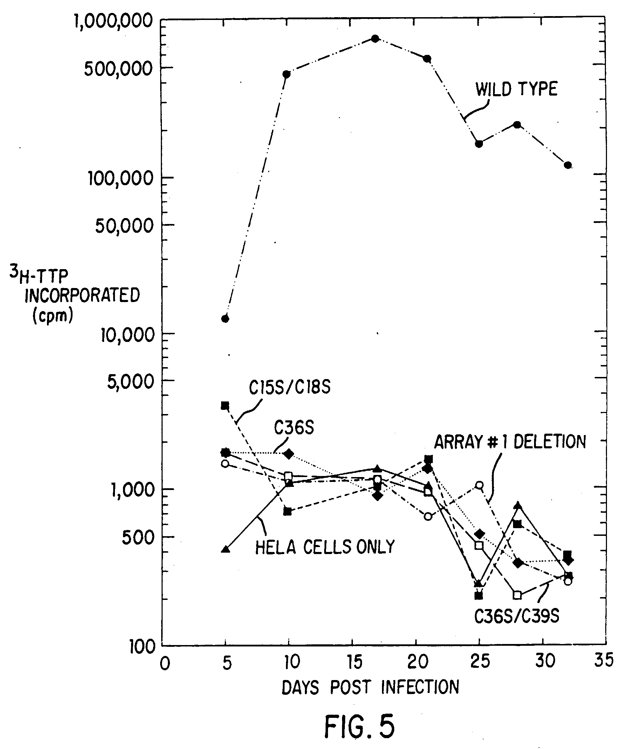

FIGURE 5 shows the infectivity assay of supernatants from Hela cells transfected with wild type and mutant HIV-I clones. Clarified supernatants from the transfected Hela cells were adjusted for equal amounts of reverse transcriptases activity in an equal volume of inoculum. These supernatants were incubated with H9 cells for 12 h in the presence of 2 µg/mL polybrene. Nine mL of complete media containing 2 µg/mL polybrene was added to the incubations and the H9 suspensions were transferred to 25 cm² tissue culture flasks. On the days indicated, samples were removed from these H9 cultures, clarified and tested for the presence of reverse transcriptase activity. The samples tested are noted on the figure.

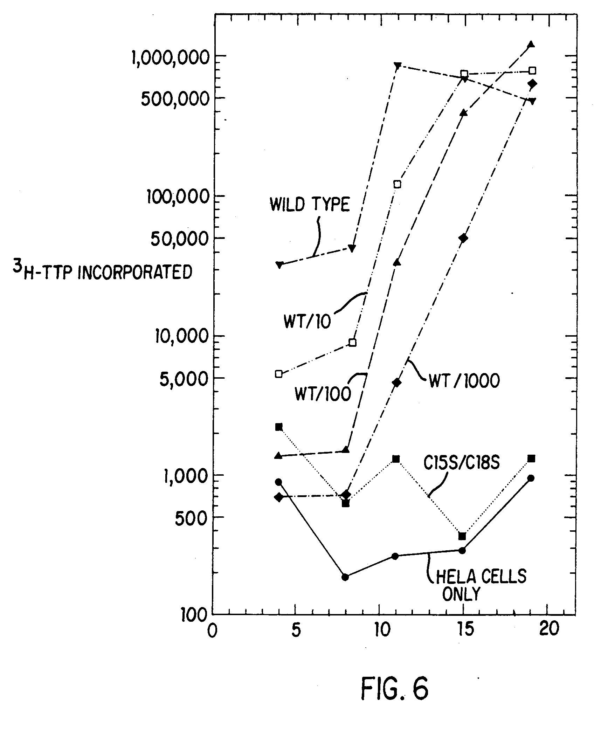

FIGURE 6 shows endpoint dilution assay of wild type and the C15S/C18S mutant. One mL of clarified supernatants from Hela cells transfected with the respective clones were treated as in Figure 5. The wild type viral supernatant was also diluted 10, 100, and 1000 fold with media; the C15S/C18S mutant was undiluted. On the days indicated, samples were removed, clarified and tested for the presence of reverse transcriptase activity. The samples and dilutions tested are indicated on the figure.

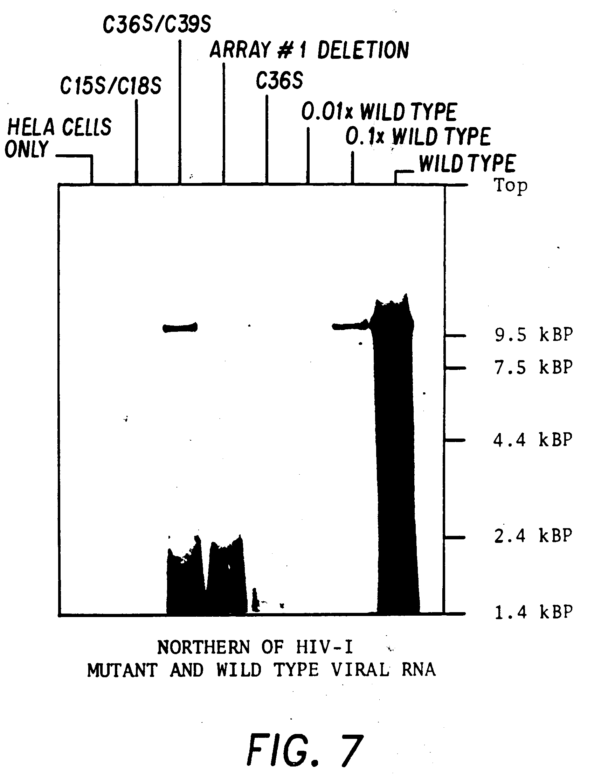

FIGURE 7 shows the northern analysis of mutant and wild type HIV-I viral RNA. Supernatants were treated as described in Figure 4 and processed for Northern analysis. A nick translated, full length HIV-I clone was used to probe for genomic RNA. Samples are as indicated on the figure.

According to the present invention, the infectivity of HIV-1, which contains at least one copy of the following amino acid sequence:
-Cys-X-X-Cys-X-X-X-X-His-X-X-X-X-Cys-
(hereinafter referred to as the cysteine array), can be drastically reduced or completely eliminated by generating mutants that lack some or all of the invariant residues which constitute part of a vital protein structure common to all retroviruses and involved in the selection and packaging of genomic viral RNA into infectious virus particles.

The present invention is directed to non-infectious human immunodeficiency virus 1 (HIV-1). This virus contains two highly conserved cysteine arrays. As described in Example 2 below, mutations have been made in both cysteine arrays of HIV-1 and the results indicate that alteration of either cysteine array results in non-infectious or very poorly infectious particles.

Examples of specific retroviruses for which the sequence data of the arrays are available may be seen in Table 1 below.

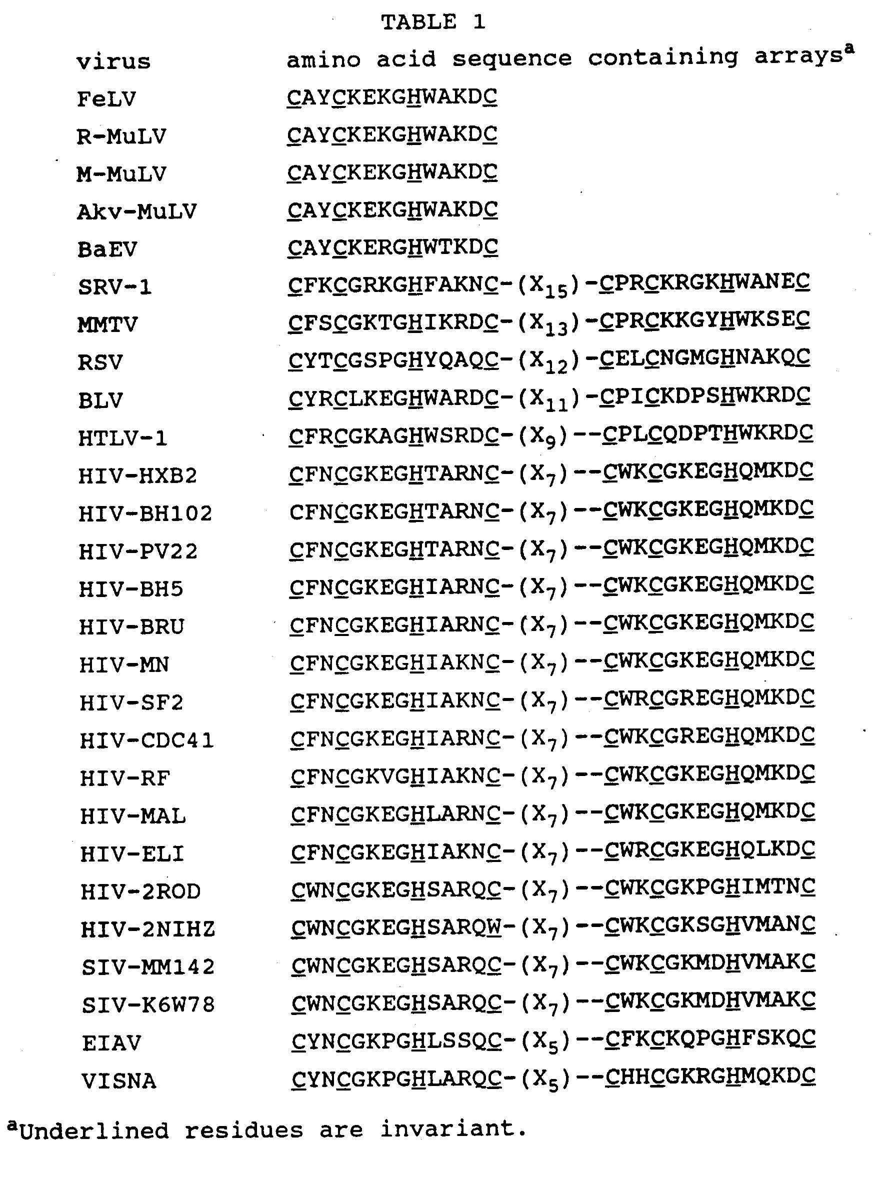

The invariant nature of the cysteine array was first described in 1981 (L. Henderson et al (1981) J. Biol. Chem. 256, 8400-8406); however, previous to now, its vital importance to viral replication has not been known.

Methods for constructing non-infectious retro-viruses having substitutions, additions or deletions of invariant and variable residues within the cysteine array include, for example, oligonucleotide-directed mutagenesis (M. Zoller et al (1982) Nucl. Acids Res. 10, 6487-6500) as described in Example 1 below. In addition, other methods whereby mutants can be constructed include cassette mutagenesis (J. Wells et al (1985) Gene 34, 315-323), and sodium bisulfite mutagenesis (R. Pine et al (1987) Methods Enzymol. 154, 415-430) of single stranded DNA regions prepared by the gapped duplex method (W. Kramer et al (1987) Methods Enzymol. 154, 350-367), and polymerase chain reaction (A. Hemsley et al (1989) Nucl. Acids Res. 17, 6545-6551).

The non-infectious retroviruses of the present invention can be used in the preparation of vaccines. As noted above, the infectivity of the mutants is drastically reduced or completely eliminated, as the mutants do not contain the normal complement of viral RNA; however, the surface components of the mutants are identical to the surface components of live virus containing viral RNA. Accordingly, antibodies can be raised against the surface components of the mutants and the antibodies thus generated will be effective against the live virus having corresponding surface features. Thus, the non-infectious retroviruses can be used in the design of vaccines which are safe to use and effective against any retrovirus.

With respect to the HIV-1 mutants, it is believed that there are epitopes contained in the mutant virions (other than those from gp120) that have not previously been seen in other studies with viral protein subunit, peptide, heat or chemically inactivated virion inoculations. These are expected to elicit strong antibody responses and be less variable than the *env* epitopes that are currently under study. Thus, a wider range of HIV-1 strains are expected to be susceptible to immunization against these common epitopes.

To decrease the likelihood of infectious revertants, additional alterations can be introduced into a retroviral genome that will yield non-infectious particles and these particles will still remain essentially native-like in structure. These include the following: removal of the packaging or psi region of the genome, located in the 5' portion of the genome, prevents recognition and packaging of genomic RNA; alteration of the proviral 3' U3 region creates virions that, even if they do contain small amounts of RNA, could not initiate transcription upon subsequent infection cycles; removal of the gene coding for the integrase (IN) protein prevents subsequent integration of proviral sequences into the host cellular genome; and engineering multiple alterations into the clone decrease the chances of reversion.

The non-infectious retroviruses of the present invention can also be used in the development of effective diagnostic procedures for all known retroviruses as well as the development of effective therapeutic agents. As indicated above, the present invention has enabled the determination of the biological role of the cysteine array, which has been found to be involved in the selection and packaging of genomic viral RNA into infectious virus particles. Because the structural features of all known retroviruses differ from each other except for this component, which is common to all, any method which detects the cysteine array or intervenes with its functioning can be used to detect all known retroviruses. Thus, for example, the cysteine array presents a target for devising reagents which can be used in the development of effective diagnostic procedures, as well as the development of therapeutic agents.

For example, the development of mutant HIV viruses that can, for only one round, normally infect and integrate into host genomic DNA is expected to be of great utility in AIDS studies and in large scale production of virions for reagent purposes. This procedure can, of course, be adapted to any intact retroviral clone. Since, for example, the mutant HIV-1 virions do not package their genomic RNA due to the inability of the nucleocapsid protein domain to recognize its genome, sequential transfection of a cysteine array mutant clone and a clone with an SV-40 (or other suitable) promoter linked to *gag* or *gag*-*pol* genes will allow for complementation of mutant virus proteins, resulting in the packaging of the mutant retroviral genome. The complementing SV-40/*gag* genome will not be packaged as there is no packaging signal. The resulting virus, with normal *gag* proteins and mutant genomic RNA, will be able to undergo one round of infection and integrate its provirus into susceptible cells, such as peripheral blood leukocytes (PBLs) or established cell lines. These cells will then have stably integrated mutant genome that produces mutant virions. These mutant virions being expressed from this stable integration are once again unable to package their RNA genome since the only nucleocapsid proteins being produced in these cells are altered in the sensitive cysteine array region.

In another embodiment, mutant viral strains that are infectious for only one cycle are introduced into PBLs isolated from a patient and subsequently returned back into the patient blood stream. The infected PBLs will then express defective virus that will act as a constant source of immunogen. Over a period of time, the PBLs will die out and the mutant virions, since they are non-infectious, will be cleared from the patient.

In still another embodiment, H-9 or other suitable cells containing a stably integrated mutant provirus are obtained in a similar fashion. Such cells are then a continuous source of mutant, non-infectious virus particles, resulting in increased safety in the laboratory or in diagnostic tests.

In yet another embodiment, the mutant viruses are utilized for the purposes of reagents in basic retroviral research. The viruses have the added safety of being non-infectious, yet they appear to be normal in all respects except for the absence of genomic RNA. The mutant viruses can be used as reagents for detecting antibodies in infected individuals as is now the case with kits using retroviral *env* component proteins or peptides. It is expected that there are other, less variable, epitopes present on these virions that will allow for the detection of a wider variety of viral strains.

In still another embodiment, the mutant viruses are used in the development of anti-retroviral agents. Due to the exquisite sensitivity of the conserved amino acid region to alteration, the development of chemical reagents targeted toward the amino acid sequence is expected to be of great utility in combating a retroviral infection. It will also prevent the further spread of retroviruses if used prophylactically. These reagents will bind to this region of the protein and render it useless in recognizing its genomic RNA, thus creating non-infectious virus.

Additionally, due to the highly conserved nature of these arrays found in all retroviral nucleocapsid proteins, screening procedures to detect any and all retroviruses can be developed. These screening procedures will be useful in identifying retroviruses that have been implicated in a number of diseases. The screening procedures may include detection of common nucleotide sequences found in the gag gene coding for this conserved cysteine array or the detection of the nucleocapsid proteins containing this common region.

The present invention will be illustrated in detail in the following Examples. These Examples are included for illustrative purposes and should not be considered to limit the present invention.

### EXAMPLE 1

### Generation of Mutations in Moloney Murine Leukemia Virus Nucleocapsid Protein, p10^{gag}

### Methodology

Cell Lines: Chinese hamster ovary (CHO) cells were described previously (M. Gottesman, ed. (1985) Molecular Cell Genetics (Wylie-Interscience, New York), p. 883; A. Rein et al. (1986) Proc. Natl. Acad. Sci. USA 83, 7246-7250). The K-NRK line of rat cells transformed by Kirsten sarcoma virus (KiSV) (S. Aaronson et al (1971) J. Gen. Virol. 13, (245-252) was a gift of Steve Showalter (National Cancer Institute-Frederick Cancer Research Facility).

Bacteria: Library efficiency DH5α competent cells used for transformations involving non-M13 plasmids and DH5αF' competent cells used for transformations with M13 type plasmid constructs were obtained from Bethesda Research Laboratories. Transformation of these hosts with plasmid constructs were performed according to the manufacturers protocols.

Plasmids: All viral subclones were derived from an infectious clone of Moloney murine leukemia virus (M-MuLV) originally obtained from D. Steffen (A. Rein et al (1986) Proc. Natl. Acad. Sci. USA 83, 7246-7250). A complete infectious clone (designated pRR88) containing the M-MuLV genome in a selectable vector was constructed using 14.0-kilobase (Kb) *Eco* RI - *Xho* I fragment from a clone of M-MuLV in Charon 4A vector, and the 6.5 kb *Eco* RI - *Sal* I portion of the pGCcos3neo selectable vector (a gift from Gray Crouse, Emory University, Atlanta, GA) which is a modified version of the pSV2neo vector containing a *SAl* I site (P. Southern et al (1982) J. Mol. Appl. Genet. 1, 5177-5181); pUC118 (J. Vieira et al 1987) Meth. Enzym. 153, 3-11) was a gift of J. Vieira (Rutgers Campus, State University of New Jersey, New Brunswick ,NJ). A c-Ki-ras clone, pSW11.1 (M. McCoy et al (1984) Mol Cell. Biol. 4, 1577-1582), was a gift of M. Barbacid (National Cancer Institute-Frederick Cancer Research Facility).

Cloning and Mutagenesis Reagents: All cloning enzymes used in this work were from either New England Biolabs, or Bethesda Research Laboratories. All plasmid preparations used were cesium banded. Mutagenic oligonucleotides and the *Pst* I - *Xho* I adaptor (see below) were prepared on an Applied Biosystems 380B DNA Synthesizer. The sequences of the mutagenic oligonucleotides are listed in Table 2 below:

**TABLE 2**

| Oligo | Size | Starting Position^{a} | Sequence (5'->3') |
|---|---|---|---|
| C26S | 20 | 2117 | CGA TCG CGA CCA GTC TGC CT |
| C26S/C29S | 27 | 2117 | CGA TCG CGA CCA GTC TGC CTA CTC CAA |
| C29S | 20 | 2126 | CCA GTG TGC CTA CTC CAA AG |
| Y28S | 20 | 2123 | CGA CCA GTG TGC CTC CTG CA |
| W35S | 20 | 2144 | AGA AAA GGG GCA CTC GGC TA |
| C39S | 20 | 2156 | CTG GGC TAA AGA TTC TCC CA |

| | | | |
|---|---|---|---|
| ^{a}Nucleotide position of M-MuLV sequence (T. Shinnick et al (1981) Nature (London) 293, 543-548) | | | |

Mutagenesis: Mutagenesis was performed in a pUC118 subclone of Moloney MuLV (designated pRR89) which extended from the *Xho* I site (nucleotide 1560 [T. Shinnick et al (1981) Nature (London) 293, 543-548]) to the *Sal* I site (nucleotide 3705), using an oligonucleotide as a single-stranded adaptor or "bandaid" between the Moloney *Xho* I site and the pUC118 Pst I site. The adaptor was 5' phosphorylated (M. Zoller et al (1982) Nucl. Acids Res. 10, 6487-6500) and had the following sequence: 5'-TGAGGCTGCA-3'. The ligation was performed with a 500-fold molar excess of the adaptor over the vector and the 2145 bp insert (ligation consisted of 0.1 pmole of 2145 pb insert and 0.01 pmole of puC118 vector). Single-stranded DNA was obtained by superinfecting TG1 bacteria containing the Moloney subclone with M13K07 (a gift of J. Vieira) as described (J. Vieira et al (1987) Meth. Enzym. 153, 3-11). Other plasmid vectors from which one can recover single stranded DNA for mutagenesis templates include the M13mp series, pUc100 series, or Bluescript vectors. Oligonucleotide-directed mutagenesis was performed using reagents and procedures supplied by Amersham Corp. (Arlington Heights, IL). In each case, candidate mutant plasmids were analyzed by chain termination sequencing of single-stranded DNA (F. Sanger et al (1977) Proc. Natl. Acad. Sci. USA 74, 5463-5467) using Sequenase (U.S. Biochemical Corp., Cleveland, OH) between the *Xho* I site and the *Sac* I site at nucleotide 2554. Templates for sequencing were constructed by insertion of mutant 1003 bp *Pst* I - *Sal* I site at nucleotide 2554. Templates for sequencing were constructed by insertion of mutant 1003 bp *Pst* I - *Sal* I fragments into homologous restriction sites of M13mp18. Oligonucleotides for verifying the mutant 994 bp *Xho* I - *Sac* I sequences were prepared as described above and are listed in Table 3 below:

**TABLE 3**

| Oligo | Size | Starting Position^{a} | Sequence (5'->3') |
|---|---|---|---|
| A | 26 | 2576 | AAC CCT CAA AGT CGG GGG GCA ACC CG |
| B | 26 | 2366 | TGA TAA GTC TGC CTG GGT CCA AGG GG |
| C | 26 | 2456 | CAC CCA CTC TTT CCT CCA TGT ACC AG |
| D | 26 | 2186 | AGG ACC TCG GGG ACC AAG ACC CCA GA |
| E | 21 | 3656 | TGC CCC AGC CCT GGG GTT GCC |
| F | 21 | 3356 | GCC CTC GGC CAA GAA AGC CCA |
| G | 21 | 2756 | AGC AAC CTC TAC CCC CGT GTC |
| H | 21 | 3056 | TGC CTT TTT CTG CCT GAG ACT |

| | | | |
|---|---|---|---|
| ^{a}Nucleotide position of M-MuLV sequence (T. Shinnick et al (1981) Nature (London) 293, 543-548) | | | |

This 994 BP *Xho* I - *Sac* I fragment, containing the wild-type sequence except for the desired change, was then used in the reconstruction of the intact M-MuLV genome. Mutant virus constructions were performed by ligating the mutant 994 Bp *Xho* I - *Sac* I fragment made from pRR89 (see above) with the 5.1 kb *Sac* I - *Cla* I and the 13 kb *Xho* I - *Cla* I portions from the infectious clone (PRR88). To ensure that the reconstructed mutant plasmids contained no defects other than the desired mutation, the 994 BP *Xho* I - *Sac* I mutant fragment in each case was replaced with the corresponding fragment from wild-type pRR89. The resulting plasmids (constructed as described above) gave rise to fully infectious M-MuLV upon transfection into hamster cells, based upon transient infectivity assays. Mutations are designated as follows: a mutation changing the cysteine codon at position 26 of p10^{gag} to a serine codon is named C26S.

Transfection and Tissue Culture Maintenance: Viral DNA constructs were introduced into Chinese hamster ovary (CHO) and the K-NRK line of rat cells transformed with Kirsten sarcoma virus by calcium phosphate coprecipitation (F. Graham et al (1973) Virology 52, 456-467) and 48 h after transfection, cells containing the viral constructs and thus neomycin resistance were selected using 400 µg/ml G418 (Geneticin, Bethesda Research Laboratories). CHO cells were grown in alpha modified Eagle medium in the presence of 5% fetal calf serum, 400 µg/ml G418 and 0.3 µM dexamethasone in an atmosphere supplemented with 5% Co₂ at 37°C. K-NRK cells were carried in Dulbeccos modified Eagle medium with 10% fetal calf serum under identical conditions.

Virus Assays: Infectivity and reverse transcriptase assays were as described (R. Bassin et al (1971) Nature (London) 229, 564-566; A. Rein et al (1979) J. Virol. 29, 494-500; A. Rein (1982) Virology 120, 251-257; B. Gerwin et al (1979) J. Virol 31, 741-751). All infectivity assays except for SL focus assay were performed in NIH/3T3 cells. In all experiments in which virus was harvested, the cells were treated with 3 x 10⁻⁷ M dexamethasone (Sigma, St. Louis, MO) for several days before harvest.

Isolation of Virus and Viral RNA: Cultures of cells transfected with mutant and wild type viral constructs were grown to approximately 80% confluency in 150 cm² flasks. Cells were fluid changed with 35 ml of their respective media (see above) and incubated overnight. Virus was harvested by centrifugation through a 4 ml cushion of 20% sucrose in diethyl-pyrocarbonate-treated phosphate buffered saline (R. Dulbecco et al (1954) J. Exp. Med. 99, 167-182) at 25,000 RPM for 2.5 hr in a Beckman SW-27 rotor at 4°C.

Protein Immunoblotting and Immunoprecipitations: Viral proteins were fractionated by NaDodSo₄-PAGE using 10-20% acrylamide gradient gels and transferred to diazotized paper as described (J. Symington et al (1981) Proc. Natl. Acad. Sci. USA 78, 177-181). Goat antiserum to p30^{gag} (R. Versteegen et al (1980) J. Virol. 33, 983-992) was used and the antigen-antibody complex was visualized with ¹²⁵I-Protein G (Amersham Corp.). Immunnoprecipitations using Protein A Sepharose (Pharmacia) were performed according to previous procedures (A. Schultz et al (1983) J. Virol. 46, 355-361; A. Schultz et al (1979) J. Virol. 30, 255-266). Antisera to the major *gag* protein (P30^{gag}) and to the *env* protein gp70 were supplied by Dr. Alan M. Schultz (Laboratory of Molecular Virology and Carcinogenesis, NCI Frederick Cancer Research Facility, Frederick, Maryland). Densitometric analysis of autoradiograms was performed using a Biomed Instruments Soft Laser Scanning Densitometer model SL504.

Nucleic Acid Hybridization: RNA was isolated from virus particles as described (M. Bender et al (1987) J. Virol. 61, 1639-1646). Viral RNAs were separated on denaturing agarose gels (T. Maniatis et al (1982) Molecular Cloning A Laboratory Manual (Cold Spring Harbor Laboratory, Cold Spring Harbor, New York) transferred to nitrocellulose and hybridized with ³²P-labeled nick-translated probes as described (T. Maniatis et al (1982) Molecular Cloning A Laboratory Manual (Cold Spring Harbor, New York), P. Thomas (1980) Proc. Natl. Acad. Sci. USA 77, 5201-5205). Hybridized filters were washed three times in 2xSSC, 0.01% NaDodSO₄, at 42°C for 5 min, two times in 1xSSC, 0.01% NaDodSO₄ at 54°C for 20 min, and two times in 0.5xSSC at 54°C for 5 min (1xSSC is 0.15 M NaCl, 0.015 M sodium citrate).

### Results

As described above, site-directed mutagenesis was performed, creating five point mutations individually changing each of the underlined amino acids to serine as follows: 
Cys-Ala-Tyr-Cys-Lys-Glu-Lys-Gly-His-Trp-Ala-Lys-Asp-Cys.
26 27 28 29 30 31 32 33 34 35 36 37 38 39
 In addition, one double mutant encoding serine at both position 26 and position 29 were generated.

Intact Moloney MuLV genomes containing these mutations were placed in the selectable vector pGCcos3neo. The mutants were initially transfected into both hamster and mouse cells. However, the mutants frequently reverted to wild type in mouse cells, presumably by recombination with endogenous MuLV sequences (J. Colicelli et al (1987) Virology 160, 518-522). Due to this problem, the experiments described in this example were performed in hamster cells.

Mass cultures of neo' cells were tested for virus particle production by several types of assays. As is shown in Table 4 below, culture fluids were found to contain particle-associated reverse transcriptase activity at essentially the same level as the control culture transfected with the wild-type parental plasmid.

**TABLE 4**

| Properties of p10^{gag} mutants | | | | |
|---|---|---|---|---|
| Virus | p30* | RT^{†} | Infectivity | |
| | | | FIU/ml^{‡} | CPFU/ml^{§} |
| C26S | 27 | 59 | <1x10° | <1x10° |
| C29S | 23 | 52 | <1x10° | <1x10° |
| C26S/C29S | 26 | 32 | <1x10° | <1x10° |
| Y28S | 38 | 86 | <1x10° | 9x10² |
| W35S | 19 | 23 | <1x10° | 2x10° |
| C39S | 29 | 63 | <1x10° | 3x10° |
| Wild type | 27 | 69 | 7x10⁵ | N.D.** |
| CHO cells were transfected with plasmids containing mutant or wild type MuLV genomes, and stable transfectants were selected with G-418. Culture fluids from these cultures were assayed as indicated. Mutants are designated as follows: C26S is a mutant in which the cysteine codon at position 26 of p10^{gag} is changed to a serine codon. | | | | |

| | | | | |
|---|---|---|---|---|
| ∗p30, protein immunoblotting results (see Fig. 1A) expressed in arbitrary densitometric units. | | | | |
| ^{†}RT, reverse transcriptase activity, pmol of [H³]TMP incorporated per ml of culture fluid (B. Gerwin et al (1979) J. Virol. 31, 741-751). | | | | |
| ^{‡}FIU/ml, focus-inducing units/ml in the S⁺L⁻ focus assay (R. Bassin et al (1971) Nature (London) 229, 564-566). | | | | |
| ^{§}CPFU/ml, complementation plaque-forming units/ml (A. Rein et al (1979) J. Virol. 29, 494-500). | | | | |
| **N.D., not determined. | | | | |

However, the culture fluids were negative in the UV-XC test and the S⁺L⁻ focus assay, which both measure the concentration of MuLV particles capable of efficient replication in mouse cells. They were also tested in the complementation plaque assay. In this test, a complementing "helper" virus is added to the assay plate; any particles capable of initiating even a single round of infection in mouse cells will register in this assay (A. Rein et al (1979) J. Virol. 29, 494-500). All of the mutants except Y28S were at least five orders of magnitude less efficient than wild type in introducing the M-MuLV genome into target cells (CPFU value) as determined in this test. The single point mutation in the Y28S mutant also produce virus incapable of continuous rounds of infection and replication (FIU value), however, this virus was capable of infecting cells with an efficiency about three orders of magnitude less than that of the wild type virus in the CPFU assay. Thus, the mutations allow the production of virus particles containing active reverse transcriptase, but these virions are totally noninfectious in the case of five of the mutants and only poorly infectious in the case of Y28S. These results strongly support the hypothesis that the amino acids in the finger-like sequence are crucial for virus replication.

The particles were also examined by protein immunoblotting, using antiserum against the major core protein p30^{gag}. Figure 1A shows the results obtained with mutant C39S (lane 1) as well as with positive and negative controls. As can be seen, C39S particles give profiles identical to that of the wild type control. Essentially identical results were obtained with all of the mutants. These results confirm that the mutations do not impair virus particle production. Further, they show that the viral protease is functioning normally in the mutants, cleaving the *gag* polyprotein precursor to the mature *gag* proteins. In other tests of the cultures containing the mutant MuLVs, normal-looking budding particles were seen by electron microscopy of thin sections, and normal levels of *env* proteins were detected by radioimmunoprecipitation.

The virus particles were also tested for the presence of viral RNA. RNA was isolated from the particles and was tested by hybridization for the presence of viral sequences. Equal quantities of virus (as assayed by protein immunoblotting for p30^{gag}) were loaded onto each lane. As shown in Figure 1B, none of the mutants except Y28S contained detectable viral RNA. Lanes 9 and 8 contain successive 10-fold dilutions of the wild-type control RNA; this calibration indicates that Y28S particles contain approximately 1/10 as much viral RNA as wild type, while the other mutants contain less than 1/50 of the wild-type level. These results show that the mutations prevent encapsidation of the viral RNA.

The mutations described herein are in a protein-coding region of the MuLV genome. In principle, the failure of the mutant particles to package viral RNA could reflect either an alteration in the viral protein, rendering it unable to function in recognizing or packaging the viral RNA; or an alteration of "Packaging" sequences in the RNA itself, making it unrecognizable by the viral proteins involved in encapsidation. Therefore, the ability of the mutants to package a second RNA, known to be packaged with high efficiency by wild-type M-MuLV proteins, was tested. Plasmids containing the mutant or wild-type MuLV genomes were transfected into the K-NRK rat cell line transformed by KiSV, and stable transfectants were isolated. The particles produced by these cultures were then analyzed for KiSV genomic RNA by hybridization. The rescue depends upon the ability of the viral *gag* precursor polyprotein to specifically recognize and complex with the Kirsten-ras genomic RNA. As shown in Figure 2, the mutant particles all contain less KiSV RNA than the wild-type control; it was estimated from lanes 8 and 9 that the deficiency in KiSV RNA packaging ranges from 1/30 (mutants C26S and C26S/C29S) to 1/2 (mutant Y28S). These data demonstrate that the mutations in the p10^{gag} coding region have impaired the ability of the viral proteins to package viral RNA.

It is useful to compare Figure 2 with Figure 1B; the mutants seem significantly more deficient in packaging MuLV RNA than KiSV RNA. This discrepancy is consistent between the different experiments, since results virtually identical to those shown in Figure 1B were obtained when the blot shown in Figure 2 was "stripped" by heating to 100°C and then rehybridixed with an MuLV-specific probe.

The fluids analyzed in Figure 2 were also tested by infectivity assays for MuLV and KiSV. As shown in Table 5 below, low levels of infectious KiSV were "rescued" by the mutant MuLVs.

**TABLE 5**

| Virus | p30* | Infectivity | | KiSV specific infectivity^{§} |
|---|---|---|---|---|
| | | FIU/ml^{†} | CPFU/ml^{‡} | |
| C26S | 14 | 2x10° | <1x10° | .00009 |
| C29S | 17 | 4x10° | <1x10° | .0001 |
| C26S/C29S | 33 | 2x10¹ | 1x10° | .0004 |
| Y28S | 37 | 2x10³ | 3x10¹ | .03 |
| W38S | 60 | 5x10² | 1x10° | .005 |
| C39S | 25 | 4x10¹ | <1x10° | .001 |
| Wild type | 44 | 7x10⁴ | 4x10⁴ | (1) |
| K-NRK cells were transfected with plasmids containing the indicated viral genomes, and stable neo^{r} transfectants were isolated. Culture fluids were assayed as indicated. | | | | |

| | | | | |
|---|---|---|---|---|
| *p30, protein immunoblotting results expressed in arbitrary densitometric units. | | | | |
| ^{†}FFU/ml, focus-forming units of KiSV/ml. (A. Rein et al (1982) Virology 120, 251-257. | | | | |
| ^{‡}CPFU/ml, complementation plaque-forming units/ml. | | | | |
| ^{§}KiSV specific infectivity, FFU:p30 ratio of mutant/FFU:p30 ratio of wild type. | | | | |

The KiSV titers were, however, much higher than the titers of MuLV particles able to infect the assay cells (i.e., CPFU). The ratio of mutant to wild type CPFU values is the same for the data given in Tables 4 and 5 showing the reproducibility of these results. Significantly, the specific infectivities of the KiSV rescued by the mutants (shown in the far right column of Table 5) were far lower than the KiSV RNA content of these particles (Figure 2). The significance of these quantitative comparisons is discussed below.

The foregoing results indicate that the mutant particles are deficient in their ability to package viral RNA. It was considered important to determine whether this deficiency reflected an inability to specifically recognize and package viral RNA, or a total loss of the capacity to incorporate RNA into virus particles. Therefore, the particles produced by the mutants were tested for the presence of RNA. Cultures producing mutant or wild-type virus were labeled for 12 hr with ³H-uridine. The particles produced by these labeled cultures were then banded in isopycnic sucrose gradients. Three representative gradients are shown in Figure 3: a peak of radioactivity is present at 1.16 g/ml in fluids containing wild-type or C26S mutant MuLV, while no peak is observed in fluids from the control cells transfected with the plasmid vector alone. Essentially similar results were obtained with all of the mutants. In order to verify that the peak of radioactivity at 1.16 g/ml represents RNA within virus particles, its sensitivity to digestion with pancreatic ribonuclease was examined. As seen in Table 6 below, it was found that at least 50% of the radioactivity in the mutant viral peaks remained acid-precipitable after direct exposure to either 100 µg/ml RNase or 1% Triton X-100, but that they were rendered completely acid-soluble if they were treated with 1% Triton X-100 before RNase digestion. ("Triton" is a registered trade mark.)

**TABLE 6**

| ³H-Uridine Labeling of Viral RNA | | | | |
|---|---|---|---|---|
| | Untreated Control^{a} | TRITON-X 100^{b} | RNAse^{c} | RNAse TRITON-X 100 |
| C26S | 504 | 281 | 266 | 26 |
| C29S | 482 | 260 | 230 | 44 |
| C26S,C29S | 258 | 112 | 154 | 40 |
| Y28S | 394 | 188 | 183 | 34 |
| W35S | 178 | 108 | 102 | 50 |
| C39S | 334 | 172 | 216 | 47 |
| Wild Type | 687 | 390 | 367 | 71 |
| pGCcos3neo | 94 | 58 | 52 | 40 |

| | | | | |
|---|---|---|---|---|
| ^{a}Table shows TCA-precipitable CPM remaining after the indicated treatments of sucrose-banded virus. | | | | |
| ^{b}1% Triton-X 100. ("Triton" is a registered trademark.) | | | | |
| ^{c}100 µg/mL RNAse A. | | | | |

These results indicate that the mutant particles do contain RNA; thus, the mutations in p10^{gag} seem to have destroyed the specificity of the viral proteins for viral RNA, rather than the ability of the particles to take up RNA *per se*.

The point mutations obtained herein were all in an extremely highly conserved sequence which bears some resemblance to the metal-binding domains ("zinc fingers") found in a number of eukaryotic transcription factors. The present inventors found that each mutant gives rise to virus particles, but that these particles lack viral RNA. Since the particles do contain some RNA, the mutations have eliminated the specificity with which the viral proteins normally recognize and encapsidate the viral genome. Thus, the finger-like sequence motif in the p10^{gag} appears to play an essential role in this recognition process.

The above-described experiments have shown that in the case of the example virus (M-MuLV), the cysteine residues in these structures are absolutely required for infectious and replicating virus, and the present inventors conclude that all retroviruses will have a similar requirement for these residues. Since the histidine residue is also invariant in the examples given in Table 1, it is concluded that this residue is also required for infectious replicating virus. It is concluded that each of these residues functions in an as yet unspecified manner to enable the *gag* precursor polyproteins to specifically complex with viral RNA, however, additional functions are not excluded.

The methods of site directed mutagenesis to incorporate specific site, deletion and addition mutations, have been used herein to study the function of the invariant residues in the array of the example virus. It is concluded that similar mutations altering residues in the invariant arrays of any retrovirus will generate mutant forms of that virus with properties similar to those of the example given herein. Thus, for any retrovirus, it will be possible to mutate invariant residues in the arrays and produce mutant forms of the virus that are normal in all respects but do not contain significant levels of genomic viral RNA and have drastically reduced or not infectivity. Such mutants are expected to have important applications as immunogens or as sources of mature viral proteins without accompanying biological hazard.

### EXAMPLE 2

### Generation of Mutations in the gag Gene Coding for the Nucleocapsid Protein of HIV-1

Mutations were made in the *gag* gene coding for the nucleocapsid protein of HIV-1 using the methods described in Example 1 above. These mutations, introduced by the technique of oligonucleotide directed mutagenesis, are shown in Table 7 below.

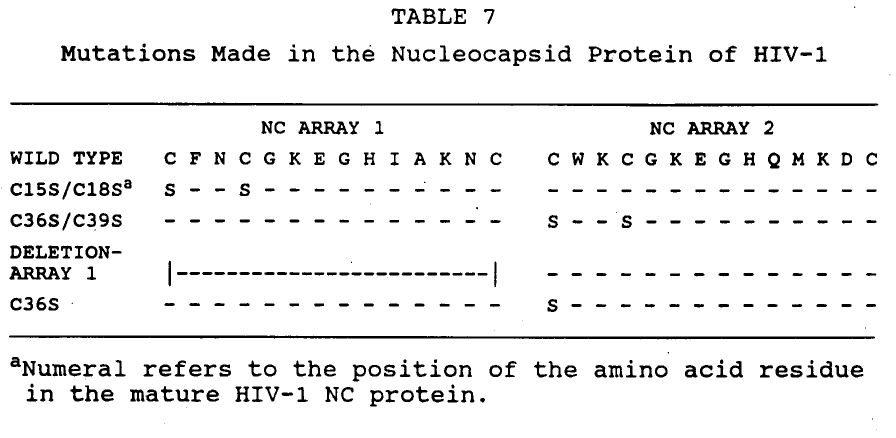

The complete mutant HIV-1 clones were transfected into HeLa cells using CaPO₄ precipitates and virus was harvested three to five days after transfection. The resulting mutant viruses were characterized by various immunochemical, protein, nucleic acid, and infectivity methods.

Mutant viral supernatants were tested for the presence of p24, using the DuPont p24 antigen capture kit. Also, the presence of reverse transcriptase (RT) activity was examined by following the incorporation of ³H-TTP into oligo-dT-poly(A) primer-template complexes. These results are shown in Table 8 below.

**TABLE 8**

| Properties of HIV-1 Mutants | | | |
|---|---|---|---|
| Mutant | p24^{CA} Content^{a} (ng/mL) | 'RT Activity^{b} ³H-TTP incorporated (cpm) | Ratio^{c} RT/p24^{CA} |
| C15S/C18S | 20.9 | 19,700 | 942 |
| C36S/C39S | 10.1 | 8,800 | 871 |
| Array #1 deletion | 7.3 | 10,500 | 1,438 |
| C36S | 9.6 | 12,000 | 1,250 |
| Wild Type | 10.4 | 15,500 | 1,490 |

| | | | |
|---|---|---|---|
| ^{a}The p24^{CA} content of clarified supernatants from Hela cells transfected with mutant and wild type HIV-I clones was tested using the DuPont HIV p24 Core Antigen ELISA kit. | | | |
| ^{b}Reverse transcriptase activity was determined by precipi tating 0.5 mL of clarified culture fluid with 0.25 mL of 50% polyethylene glycol (8000 MW) in 0.5 M NaCl. Precipitates were pelleted by centrifugation and dissolved in TNE buffer. Reverse transcriptase assays were performed on 1/5 of each sample as described (A. Hoffman et al (1985) Virology 147, 326-335). | | | |
| ^{c}Ratio of p24^{CA} (ng/mL)/RT activity (cpm) | | | |

The amounts of virus produced was quite variable which is believed to reflect the inherent variabilities in the transfection procedure. An average of 12 ng of p24 CA protein was detected per mL of supernatant, five days after transfection, from a 24 hour harvest of 30 mL of supernatant from a confluent 150 cm³ monolayer of HeLa cells. The p24 antigen capture results and RT activities showed reasonable agreement, indicating that the mutants had normal amounts of RT per virus particle.

For all of the following experiments, each supernatant was adjusted for constant amounts of virus based on the reverse transcriptase data. Immunoblotting was performed and proteins were visualized by probing with monoclonal antibody to p24 (Figure 4). It appeared that all of the mutant virions process their *gag* gene products identically to that of wild type virus, yielding mature CA protein (p24). This indicated that the viral protease was being expressed and was functioning properly. It is noted further that there were some uncleaved *gag* precursors detected by this antisera, in the wild type as well as in these mutants. Thus, it appeared that the mutant HIV-1 virions had functional *gag* and *pol* genes as seen from the Western assay shown in Figure 4 and the reverse transcriptase (RT) data shown in Table 8.

The infectious potential of these mutants was assayed by taking clarified supernatants from the transfected HeLa cells, adjusting them for equal reverse transcriptase activities, and incubating them with H-9 cells for 12 hours in the presence of 2 µg/mL polybrene. The H-9 cells were carried as usual with the addition of 2 µg/mL polybrene. The production of virus was determined by following reverse transcriptase activity over time. Supernatants from HeLa cells transfected with the wild type clone, contained infectious virus as demonstrated by a rise in H9 cell supernatant RT activity, five days post-infection (see Figure 5). Supernatants from HeLa cells transfected with the mutant HIV-1 clones produced virus that was not infectious as determined by this analysis. Even after 32 days, HeLa supernatants containing mutant virus failed to induce RT activity in H9 cells. Similar cultures were analyzed 44 days post-infection, and the mutant viruses still showed no indication of infection. The same results were seen when these supernatants were applied to human peripheral blood lymphocytes. An end-point dilution assay indicates that the mutants are at least 1000, and possibly even 10,000 times less infectious than an identical amount of wild type virus (based upon reverse transcriptase activities). As shown in Figure 6, wild type virus from Hela cell culture fluids, even after being diluted 1000 fold, is still able to productively infect H9 cells and human PBLs. Undiluted mutant, C15S/C19S, is still unable to productively infect H9 cells or human PBLs.

In an attempt to detect RNA in these mutants, Northern analysis was performed on the supernatants. The samples were adjusted for equal amounts RT activity, as judged by the ³H-TTP incorporation data (see Table 8 above). Viral RNA was isolated and separated by electrophoresis on a formaldehyde gel. The RNA was then transferred onto nitrocellulose and probed with a complete HIV-1 nick translated probe. As can be seen in Figure 7, wild type virus had a correct size band which corresponded to 9.7 kBP. The mutant virions contained reduced amounts of genomic RNA when compared to an equal amount of wild type virus. From a 1/10 and 1/100 dilution of wild type virus, both of which are detected on the exposed Northern Film, the C15S/C18S, array #1 deletion, and the C36S mutants contained approximately 2- 5% the amount of genomic RNA contained in the same amount of wild type HIV-1 particles, and the C36S/C39S mutant contained about 20% of the RNA found in wild type. It is very interesting to note that the mutants are still non-infectious, even though they have a 4-20% genomic RNA content when compared to an equal number of wild type particles. Thus, while the lack of infectivity of the mutants may be due in part to the absence of viral RNA, the results suggest that there is an additional role that is performed by this conserved cysteine array in the nucleocapsid protein, and that this function is also sensitive to these alterations. This observation has also been made in Moloney-MuLV mutants.

As shown by the above experiments, alteration of either of the two conserved cysteine arrays of HIV-1 results in non-infectious virions, phenotypically similar to those seen in Mo-MuLV which contains only one conserved array. Thus, both arrays are believed to be required for efficient recognition and packaging of homologous genomic RNA.

As has been shown, it is possible to uncouple virus assembly, budding and maturation from complexation with viral RNA by alteration of the invariant residues of arrays. It is concluded that any method of altering the chemical or biological properties of these residues will result in the production of non-infectious virus. Thus, any reagent or combination of reagents that specifically alters the biological function of these invariant residues could form the basis for the design of effective therapeutic and/or prophylactic procedures for any retrovirus.

## Claims

1. A method of reducing the infectivity of human immunodeficiency virus 1 (HIV-1) comprising introducing at least one point mutation in a cysteine array present in the nucleocapsid domain of the *gag* precursor polyprotein of said virus, said cysteine array having the sequence
-Cys-X-X-Cys-X-X-X-X-His-X-X-X-X-Cys-
wherein each X independently represents a variable amino acid, provided that said at least one point mutation does not introduce tyrosines at both of positions 1 and 4 of the cysteine array.

2. The method of claim 1, wherein said at least one point mutation is an invariant residue of said sequence.

3. The method of claim 1, wherein said at least one point mutation is a variant residue of said sequence.

4. The method of claims 1,2 or 3 wherein said at least one point mutation is such that the infectivity of said retrovirus is reduced by at least 3 orders of magnitude as measured by an infectivity assay.

5. The method of claim 4, wherein said at least one point mutation is such that the infectivity of said retrovirus is reduced by at least 4 orders of magnitude as measured by an infectivity assay.

6. The method of claims 1, 2 or 3, wherein said at least one point mutation is such that said retrovirus is rendered non-infective as measured by an infectivity assay.

7. The method of any one of the preceding claims, wherein said at least one mutation is present in two of said cysteine arrays.

8. A reduced infectivity human immunodeficiency virus 1 (HIV-1)obtainable by a method as claimed in any one of claims 1-7.

## Patentansprüche

1. Verfahren zur Reduzierung der Infektivität des menschlichen Immundefizit-Virus 1 (HIV-1), das das Einbringen mindestens einer Punktmutation in eine Zysteinreihe umfaßt, die in einem Nukleocapsid-Domain des *gag* Precursor-Polyproteins des Virus vorhanden ist und folgende Sequenz aufweist:
-Cys-X-X-Cys-X-X-X-X-His-X-X-X-X-Cys-
wobei jedes X unabhängig eine variable Aminosäure darstellt, vorausgesetzt, daß die mindestens eine Punktmutation an den beiden Positionen 1 und 4 der Zysteinreihe keine Tyrosine einbringt.

2. Verfahren nach Anspruch 1, bei dem die mindestens eine Punktmutation ein invarianter Rest der Sequenz ist.

3. Verfahren nach Anspruch 1, bei dem die mindestens eine Punktmutation ein varianter Rest der Sequenz ist.

4. Verfahren nach Anspruch 1, 2 oder 3, bei dem die mindestens eine Punktmutation in der Weise geartet ist, daß bei einer Messung durch eine Infektivitätsprüfung die Infektivität des Retrovirus um mindestens drei Größenordnungen reduziert ist.

5. Verfahren nach Anspruch 4, bei dem die mindestens eine Punktmutation in der Weise geartet ist, daß bei einer Messung durch eine Infektivitätsprüfung die Infektivität des Retrovirus um mindestens vier Größenordnungen reduziert ist.

6. Verfahren nach Anspruch 1, 2 oder 3, bei dem die mindestens eine Punktmutation in der Weise geartet ist, daß bei einer Messung durch eine Infektivitätsprüfung der Retrovirus als nichtinfektiös anzusehen ist.

7. Verfahren nach einem der vorhergehenden Ansprüche, bei dem die mindestens eine Mutation in zwei der Zysteinreihen vorhanden ist.

8. Menschlicher immundefizienter Virus 1 (HIV-1) mit reduzierter Infektivität, der mit einem Verfahren nach einem der Ansprüche 1 bis 7 hergestellt worden ist.

## Revendications

1. Une méthode pour réduire l'infectivité du virus immunodéficient humain 1 (HIV-1), consistant à introduire au moins un point de mutation dans un fragment cystéinique présent dans le domaine nucléocapsidique de la protéine précurseur de *gag* de ce virus, ce fragment cystéinique ayant la séquence
-Cys-X-X-Cys-X-X-X-X-His-X-X-X-X-Cys-
dans laquelle chaque X représente indépendamment un aminoacide variable, sous la réserve qu'au moins un point de mutation n'introduise pas de tyrosines aux deux positions 1 et 4 du fragment cystéinique.

2. La méthode de la revendication 1, dans laquelle ce au moins un point de mutation est un résidu invariant de ladite séquence.

3. La méthode de la revendication 1, dans laquelle ce au moins un point de mutation est un résidu variant de ladite séquence.

4. La méthode des revendications 1, 2 ou 3, dans laquelle ce au moins un point de mutation est tel que l'infectivité dudit rétrovirus est réduite d'au moins trois fois quand elle est mesurée par un essai d'infectivité.

5. La méthode de la revendication 4, dans laquelle ce au moins un point de mutation est tel que l'infectivité dudit rétrovirus est réduite d'au moins 4 fois quand elle est mesurée par un essai d'infectivité.

6. La méthode des revendications 1, 2 ou 3, dans laquelle ce au moins un point de mutation est tel que ledit rétrovirus est rendu non infectieux quand il est mesuré par un essai d'infectivité.

7. La méthode selon l'une quelconque des revendications précédentes, dans laquelle cette au moins une mutation est présente dans deux desdits fragments cystéiniques.

8. Un virus immunodéficient humain 1 (HIV-1) à infectivité réduite tel qu'il peut être obtenu par une méthode selon l'une quelconque des revendications 1 - 7.
